# EUROPEAN PATENT SPECIFICATION

(11) **EP 2 134 273 B1**
(45) Date of publication and mention of the grant of the patent: **02.12.2015**
(21) Application number: 08712640.5
(22) Date of filing: 27.02.2008
(51) Int. Cl.: A61B 17/425, A61B 17/43

(54) **SUITABLE APPARATUS FOR PRECISELY AND REPRODUCIBLY TRANSFERRING A FERTILIZED EGG-CELL (EMBRYO) TO THE UTERUS**
GEEIGNETES GERÄT FÜR DEN PRÄZISEN UND REPRODUZIERBAREN TRANSFER EINER BEFRUCHTETEN EIZELLE (EMBRYO) IN DIE GEBÄRMUTTER
APPAREIL APPROPRIÉ POUR TRANSFÉRER DANS L'UTÉRUS AVEC PRÉCISION ET EN VUE DE LA REPRODUCTION UN OVULE FÉCONDÉ (EMBRYON)

(30) Priority: 08.03.2007 NL 2000528
(43) Date of publication of application: 23.12.2009
(73) Proprietor: Vereniging voor Christelijk Hoger Onderwijs, Wetenschappelijk Onderzoek en Patiëntenzorg, 1081 HV Amsterdam (NL)
(72) Inventor: HOMPES, Petrus Gerardus Alphonsus, 1181 PJ Amstelveen (NL)
(74) Representative: Van Breda, Jacobus
(86) International application number: PCT/NL2008/050116
(87) International publication number: WO 2008/108642

(56) References cited:
- WO-A-99/18872
- WO-A-2005/021077
- DE-U1- 8 909 141
- US-A- 4 988 481
- US-A- 5 214 968
- US-A1- 2004 059 186
- US-A1- 2005 038 315

## Description

The present invention relates to a suitable apparatus for precisely and reproducibly transferring an outside the uterus fertilized egg-cell (embryo) to the uterus.

It is known that a considerable number of women are unable to become pregnant. The cause for this may rest with the man as well as with the woman.

Many of these women have a strong desire to have children and take recourse to artificial fertilization techniques such as in vitro fertilization (IVF) and intracytoplasmic sperm injection (ICSI).

The IVF/ICSI treatment involves several phases. The first phase concerns stimulation, wherein the patient receives hormones in order to bring a number of egg-cells to maturation. This is monitored by ultrasound.

The second phase involves puncturing, whereby the various follicles are pierced with the aid of a vaginal ultrasound probe, after which their contents are aspirated. The aspirated fluid is then passed on to the embryologist, who first establishes whether the fluid contains any egg-cells. If egg-cells are present, semen will be added in vitro to effect fertilization. After 2-3 days, the egg-cells are examined for cell division, whereby several fertilized egg-cells, also referred to as embryos, may be present.

In the third phase, the obtained fertilized egg-cells, embryos, are transferred to the uterus.

According to the prior art, this transfer takes place with the aid of a syringe attached to a catheter. By means of the syringe the embryos are aspirated with the fluid and with the aid of ultrasound guiding they are transferred to the uterus. The woman may decide whether one or two embryos are transferred to the uterus, with the chance of one or two children. For this purpose a standard commercially available 1 ml syringe is used. The catheter is comprised of a thin, flexible plastic tube, in which the embryos are enclosed between two air bubbles.

In an endeavour to improve the known method so as to increase the chance of a pregnancy much more attention is given to improving stimulation protocols than to improving the embryo transfer technique.

However, various studies have shown that the chance of pregnancy resulting from IVF/ICSI is influenced very particularly by the location to which the embryo is transferred in relation to the fundus of the uterus. It has been shown that the best chance of pregnancy is afforded by a distance of 1-2 cm between the transferred embryo and the fundus (see for example M. Lambers et al. 'The position of the transfer air bubbles after embryo transfer is related to pregnancy rates', accepted for publication in Fertility and Sterility 2006).

However, the prior art technique is imprecise and not reproducible, making it difficult to determine the transfer position of the embryo. The imprecision and non-reproducibility are caused by a number of factors such as the syringe itself, the resistance of the plunger, the pressure exerted on the plunger by the physician, and possible intra-uterine resistance partly attributable to uterine contractions. Especially the varying pressures applied when ejecting the embryo from the catheter plays a considerable role in the above mentioned imprecision and non-reproducibility.

Moreover, excessive ejection pressure may damage the embryos themselves.

US 2005/0038315 A1 discloses a motorized syringe particularly useful for intra-uterine insemation.

WO 99/18872 A1 relates to a method for depositing a flattened droplet on a surface and apparatus therefore, and a pump therefore. The two part form of independent claim 1 is based on this document.

US 2004/059186 A1 discloses an insemination device with a slow-release.

US 5,214,968 relates to pipet filling and discharge device.

It is thus an object of the invention to provide an apparatus according to claim 1 and to a now-surgical method acording to claim 4, which effectively eliminates the above mentioned drawbacks, and is able to significantly increase the chance of pregnancy resulting from an IVF treatment.

To this end, the present invention provides a suitable apparatus for precisely and reproducibly transferring to the uterus an egg-cell (an embryo) fertilized outside the uterus, which apparatus is characterised in that the same is provided with a pump, a flexible tube, a motor, control electronics, a power supply and a catheter.

Surprisingly, it was shown that by using the apparatus according to the invention, the drawbacks of the prior art are effectively removed. A special feature of the electrical plump used for embryo transfer is that it is able to aspirate a very small amount of fluid of approximately 30 microlitres within an exact time limit, as well as to inject this small amount in a reproducible manner.

The person in question, usually a physician who has to transfer the embryo to the uterus, is able to do this with the aid of the present apparatus precisely and reproducibly, such that the embryo is placed in the uterus at a proven optimal distance, preferably at 1-2 cm from the fundus.

In accordance with the present apparatus, first an amount of IVF culture medium is aspirated into the catheter, then an pair bubble, and then again an amount of IVF culture medium containing one embryo, then again an air bubble, and finally another amount of culture medium. The embryos are then, as it were, enclosed in the catheter between the two air bubbles.

With the aid of ultrasound guiding the treating physician then inserts the catheter into the uterus and ensures that the end of the catheter is positioned at the desired location, preferably at 1-2 cm from the fundus of the uterus. Then the pump is switched on and the embryos are ejected slowly and evenly from the catheter.

When the embryos have being placed into the uterus at the desired distance from the fundus, the catheter is withdrawn from the uterus.

The control electronics provided in the apparatus enable the suction and pushing force of the apparatus according to the invention to be adjusted with precision. In comparison with the prior art syringe, it is possible that the chance of pregnancy may be significantly increased with the aid of the apparatus according to the invention.

An important advantage of the present apparatus is further that diverse physicians are able to use it with the same precision and reproducibility because when ejecting the embryos from the catheter using the pump, the pressure can be kept constant, which is not possible with the syringe. The difference to using a syringe is that the ejection pressure may vary depending on the person operating the syringe.

The invention will now be further explained with reference to the accompanying Figures 1-3. The invention is not limited to the embodiments illustrated in the Figures 1 and 2.
Figure 1 shows the apparatus according to the invention.
Figure 2 shows the catheter according to the invention, and
Figure 3 shows the apparatus according to the prior art.

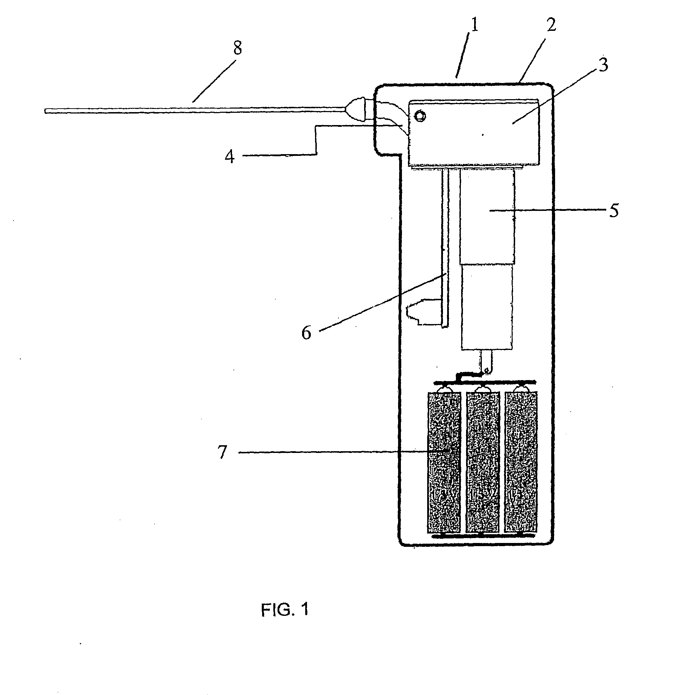

Figure 1 shows the apparatus (1) according to the invention, provided with a pump (3), a flexible tube (4), a motor (5), control electronics (6), a power supply (7) and catheter (8). It is usually that a battery serves as power supply, but the mains power system is also possible, in which case the apparatus is provided with a cord and plug.

The pump (3), flexible tube (4), motor (5), control electronics (6) and power supply (7) are accommodated in a housing (2) for easy handling of the apparatus (1) and for the protection of the various components in the apparatus (1).

Via the catheter, the embryologist will with the aid of the apparatus first aspirate in vitro a small amount of culture medium, then an air bubble, then an amount of culture medium containing one or two embryos, depending on the wish of the patient, then air again and finally again an amount of culture medium. In total, the amount of inserted fluid is only 30 microlitres.

The treating physician, usually a gynaecologist, will subsequently take the apparatus to the treatment room where the transfer of the embryo takes place.

With the aid of ultrasound guiding the treating surgeon will insert the catheter into the uterus such that its end is located at a distance of 1-2 cm from the fundus. Using the present apparatus, the contents of the catheter are then gradually, at a constant pressure ejected from the catheter such that the air bubbles, and thus the embryos, are deposited at a distance of 1-2 cm from the fundus of the uterus. Any arbitrary physician is able to carry out this operation consistently and reproducibly, with the possible result of the thus treated woman having a significantly greater chance of pregnancy.

In the apparatus (1) according to the invention, the power supply (7) drives the motor (5) bringing about the pumping action of the pump (3). Instead of batteries, it is possible to use another power source, for example the electric power mains, in which case batteries are superfluous. The suction and ejection pressure generated by the pump (3) is passed on via catheter (8), which by means of the flexible tube (4) is connected with the pump (3). It is preferred for the catheter to be removably connected with the flexible tube (4).

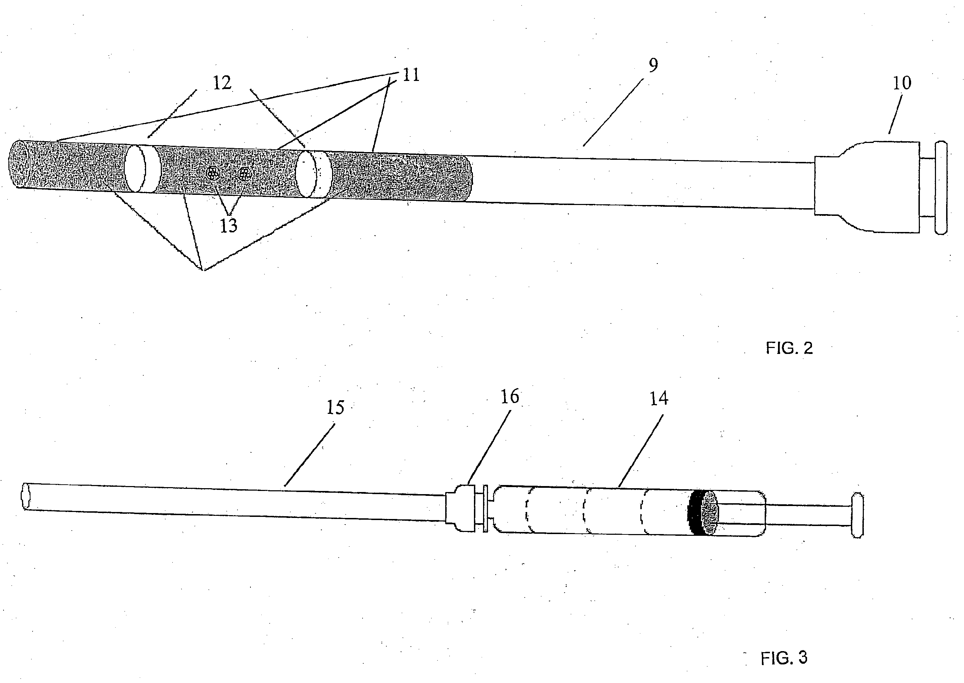

Figure 2 shows a catheter (9), whose one end is provided with a connecting piece (10) for connecting to the end of the flexible tube (4).

In Figure 2, the amount of culture medium is indicated with reference numeral (11), the air bubbles carry reference numeral (12), and the embryo(s) reference numeral (13).

The apparatus according to the prior art is represented in Figure 3. In Figure 3, the catheter (15) is connected with a syringe (14) by means of a connection (16).

With regard to precision and reproducibility, this apparatus is inferior to the apparatus according to the invention, as a consequence of which the chance of pregnancy when using such an apparatus is significantly smaller than when using the present apparatus.

It should be noted that the present invention is not limited to the embodiments shown in the Figures 1 and 2 of the present invention.

## Claims

1. Apparatus (1) suitable for precisely and reproducibly transferring an outside the uterus fertilized egg-cell, i.e. embryo to the uterus, which apparatus (1) is provided with an electrical pump (3), a flexible tube (4), a motor (5), control electronics (6), a power supply (7) and a catheter (8), **characterised in that** the pump (3), the flexible tube (4), the motor (5), the control electronics (6), and the power supply (7) are accommodated in a housing (2), and
*wherein the electrical pump (3) is able to aspirate a very small amount of fluid of approximately 30 microlitres,*
wherein the control electronics (6) *provided in the apparatus* (1) are for adjusting precisely the suction and pushing force *of the pump* (3) of the apparatus (1) and
the electrical pump (3) is for embryo transfer by
aspirating within an exact time limit a first *precise and reproducible* amount of IVF culture medium into the catheter (8),
then an air bubble, and
then again *a precise and reproducible* amount of IVF culture medium containing the embryo,
then again an air bubble, and
finally another *precise and reproducible* amount of culture medium
*such that diverse physicians are able to transfer the embryo with the same precision and reproducibility using the pump* (3) *as the pressure when ejecting the embryo is kept constant.*

2. An apparatus (1) according to claim 1, wherein the power supply (7) is a battery.

3. An apparatus (1) according to any of the preceding claims, **characterized in that** the catheter (8) is removably connected with the flexible tube (4).

4. Non-surgical method of aspiring an IVF embryo comprising the steps of providing an apparatus (1) according to any of the preceding claims,
aspirating a first amount of IVF culture medium into the catheter (8),
then an air bubble, and
then again an amount of IVF culture medium containing one or several embryo(s), then again an air bubble, and
finally another amount of culture medium.

## Patentansprüche

1. Vorrichtung (1), welche zum präzisen und reproduzierbaren Transfer einer außerhalb der Gebärmutter befruchteten Eizelle, d.h. einem Embryo, in die Gebärmutter geeignet ist, wobei die Vorrichtung (1) mit einer elektrischen Pumpe (3) versehen ist, einem flexiblen Schlauch (4), einem Motor (5), einer Steuerelektronik (6), einer Stromversorgung (7) und einem Katheter (8), **dadurch gekennzeichnet, dass** die Pumpe (3), der flexible Schlauch (4), der Motor (5), die Steuerelektronik (6) und die Stromversorgung (7) in einem Gehäuse (2) untergebracht sind, und
wobei die elektrische Pumpe (3) eine sehr kleine Flüssigkeitsmenge von ungefähr 30 Mikrolitern aspirieren kann,
wobei die in der Vorrichtung (1) vorgesehene Steuerelektronik (6) zur präzisen Anpassung der Ansaug- und Druckkraft der Pumpe (3) der Vorrichtung (1) vorgesehen ist und
die elektrische Pumpe (3) vorgesehen ist für Embryonentransfer durch
Aspirieren einer ersten präzisen und reproduzierbaren Menge von IVF-Nährmedium in den Katheter (8) innerhalb eines exakten Zeitlimits,
dann einer Luftblase, und
dann erneut einer präzisen und reproduzierbaren Menge von IVF-Nährmedium, welche den Embryo enthält,
dann erneut einer Luftblase, und
abschließend einer weiteren präzisen und reproduzierbaren Menge von IVF-Nährmedium,
so dass unterschiedliche Ärzte dazu fähig sind, den Embryo mit dergleichen Präzision und Reproduzierbarkeit unter Verwendung der Pumpe (3) zu transferieren, wobei der Druck beim Ausstoßen des Embryos konstant gehalten wird.

2. Vorrichtung (1) nach Anspruch 1, wobei die Stromversorgung (7) eine Batterie ist.

3. Vorrichtung (1) nach einem der vorstehenden Ansprüche, **dadurch gekennzeichnet, dass** der Katheter (8) lösbar mit dem flexiblen Schlauch (4) verbunden ist.

4. Nicht-chirurgisches Verfahren des Aspirierens eines IVF-Embryos, umfassend die Schritte:
Bereitstellen einer Vorrichtung (1) gemäß einem der vorstehenden Ansprüche,
Aspirieren einer ersten Menge von IVF-Nährmedium in den Katheter (8),
dann einer Luftblase, und
dann erneut einer Menge von IVF-Nährmedium, welche einen oder mehrere Embryonen enthält,
dann erneut einer Luftblase, und
abschließend einer weiteren Menge von Nährmedium.

## Revendications

1. Appareil (1) adapté au transfert dans l'utérus, de façon précise et reproductible, d'un ovule fécondé (embryon) hors de l'utérus, lequel appareil (1) est pourvu d'une pompe électrique (3), d'un tube souple (4), d'un moteur (5), de commandes électroniques (6), d'une alimentation électrique (7) et d'un cathéter (8), **caractérisé en ce que** la pompe (3), le tube souple (4), le moteur (5), les commandes électroniques (6), et l'alimentation électrique (7) sont logés dans un boîtier (2), et
la pompe électrique (3) étant apte à aspirer une très petite quantité de fluide d'environ 30 microlitres,
les commandes électroniques (6) situées dans l'appareil (1) servent à régler avec précision la force d'aspiration et de poussée de la pompe (3) de l'appareil (1), et
la pompe électrique (3) sert au transfert de l'embryon par
aspiration, dans un intervalle de temps précis, d'une première quantité, précise et reproductible, de milieu de culture de FIV dans le cathéter (8),
puis d'une bulle d'air, et
puis de nouveau d'une première quantité, précise et reproductible, de milieu de culture de FIV contenant l'embryon,
puis de nouveau d'une bulle d'air, et
enfin, d'une autre quantité, précise et reproductible, de milieu de culture de manière que divers médecins puissent transférer l'embryon avec la même précision et reproductibilité en utilisant la pompe (3) tandis que la pression lors de l'éjection de l'embryon est maintenue constante.

2. Appareil (1) selon la revendication 1, dans lequel l'alimentation électrique (7) est une batterie.

3. Appareil (1) selon l'une quelconque des revendications précédentes, **caractérisé en ce que** le cathéter (8) est relié, de façon amovible, au tube souple (4).

4. Procédé non-chirurgical d'aspiration d'un embryon de FIV, comprenant les étapes consistant à :
fournir un appareil (1) selon l'une quelconque des revendications précédentes,
aspirer une première quantité de milieu de culture de FIV dans le cathéter (8),
puis une bulle d'air, et
puis de nouveau une première quantité de milieu de culture de FIV contenant un ou plusieurs embryons,
puis de nouveau une bulle d'air, et
enfin, une autre quantité de milieu de culture.
